# EUROPÄISCHE PATENTSCHRIFT

(11) **EP 0 842 633 B1**
(45) Veröffentlichungstag und Bekanntmachung des Hinweises auf die Patenterteilung: **01.10.2003**
(21) Anmeldenummer: 97120095.1
(22) Anmeldetag: 17.11.1997
(51) Int. Cl.: A61B 1/05, A61B 1/00

(54) **Voll autoklavierbares elektronisches Endoskop**
Electronic endoscope for all autoclave use
Endoscope électronique pouvant être entièrement stérilisé à l'autoclave

(30) Priorität: 19.11.1996 DE 19647855
(43) Veröffentlichungstag der Anmeldung: 20.05.1998
(73) Patentinhaber: Henke-Sass, Wolf GmbH, D-78532 Tuttlingen (DE)
(72) Erfinder: Schulz, Dieter, 78570 Mühlheim (DE); Brunnen, Rainer, 78606 Seitingen-Oberflacht (DE); Häckl, Norbert, 88637 Leibertingen (DE)
(74) Vertreter: Fehners, Klaus Friedrich, Dipl.-Ing., Dipl.-Wirtsch.-Ing

(56) Entgegenhaltungen:
- EP-A- 0 426 063
- WO-A-93/06767
- US-A- 4 878 485
- US-A- 5 177 605
- US-A- 5 402 768
- PATENT ABSTRACTS OF JAPAN vol. 097, no. 002, 28.Februar 1997 & JP 08 280610 A (OLYMPUS OPTICAL CO LTD), 29.Oktober 1996,

## Beschreibung

Die Erfindung bezieht sich auf ein elektronisches Endoskop mit einem Halbleiter-Bildsensor in Form eines CCD-Chips und einer Filter-Anordnung, die mit dem CCD-Chip zu einer CCD-Chip-Einheit zusammengefaßt ist, zur Aufnahme der von einem Objektiv aufgenommenen Bilder und einer elektronischen Schaltung, wobei das Endoskop im wesentlichen aus einem Schaft mit einem distalen und einem proximalen Ende besteht und das Objektiv sowie die CCD-Chip-Einheit am distalen Ende angeordnet und das proximale Ende in einem den Schaft haltenden und einen Glasfaserlichtanschluß umfassenden Gehäuse gehalten ist, wobei die Bestandteile der hinter dem Objektiv angeordneten CCD-Chip-Einheit, nämlich ein Kristallfilter, ein IR-Cut-Filter und der CCD-Chip jeweils mit einem freien Zwischenraum zueinander angeordnet und in einer Metallfassung gehalten sind, die einen Bestandteil der Einheit bildet und an welche sich eine Platine mit den die Videosignale leitenden Kabeln anschließt.

Solche Endoskope, die insbesondere im medizinischen Bereich eingesetzt werden, müssen nach ihrer Verwendung und in Vorbereitung eines neuen Einsatzes sterilisiert werden, insbesondere in ihrem distalen Bereich, der in den menschlichen Körper eingeführt wird.

Unabhängig davon, wie die vorbekannten Endoskope bzgl. CCD-Chips ausgebildet und speziell innerhalb des Endoskop-Schaftes eingerichtet sind, ist es nicht möglich, diese Instrumente voll zu autoklavieren, d. h., sie halten auf Dauer der dabei mindestens einzusetzenden Temperaturhöhe von 134°C nicht stand. Der Grund dafür liegt darin, daß die CCD-Chip-Einheit, die mehrteilig ausgebildet ist und deren einzelne Bauteile miteinander verklebt sind und in einem Metall-Element gehalten werden, nach mehreren Autoklavier-Vorgängen "erblindet", und zwar dadurch, daß sich aufgrund der Temperaturerhöhung während des Autoklavierens und des sich daran anschließenden Abkühlens die Oberflächen der einzelnen Teile, insbesondere des dem eigentlichen CCD-Chip vorgesetzten Kristallfilters und des IR-Cut-Filters, in ihrer Qualität verschlechtern, da nämlich der zwischen deren jeweiligen benachbarten Oberflächen eingebrachte Kleber nicht mehr durchsichtig bleibt, sondern matt wird.

Zur Lösung des vorstehend dargestellten Problems ist es aber bereits aus der EP 0 426 063 A1 in Verbindung mit der US 5,177,605 bekannt, die einzelnen Bestandteile der CCD-Chip-Einheit, also den CCD-Chip selbst und die sich davor befindenden speziellen Filter jeweils mit einem freien Zwischenraum zueinander anzuordnen. Die Kombination eines CCD-Chips mit einem Kristallfilter und einem IR-Cut-Filter ist auch der JP 08280610A zu entnehmen, diese letztgenannte Schrift zeigt auch die Zusammenfassung der CCD-Chip-Einheit in einer festen Metallfassung.

Die in diesen Druckschriften beschriebene Ausbildung der CCD-Chip-Einheit gewährleistet zwar, daß die aneinander zugewandten Oberflächen der vorgenannten Bestandteile nicht miteinander in Berührung kommen und die jeweiligen Bestandteile deshalb nicht miteinander verklebt sein müssen und sich folglich auch keine Verschlechterung der Qualität der CCD-Chip-Einheit durch Veränderungen des Klebemittels einstellt, wenn das Endoskop den Autoklavierbedingungen ausgesetzt wird. Es hat sich allerdings in der Praxis gezeigt, daß aufgrund der unterschiedlichen Ausdehnungen der CCD-Chip-Einheit bzw. der in einer solchen Einheit zusammengefaßten jeweiligen Bauteile diese während der Durchführung des Autoklaviervorganges beschädigt werden können, beispielsweise Verformungen bzw. Risse erhalten können, speziell aufgrund einer festen Lagerung in einem Gehäusebestandteil des Endoskopes.

Es ist deshalb ein Ziel der Erfindung, ein insbesondere im medizinischen Bereich einsetzbares Endoskop zu schaffen, das voll autoklavierbar ist, also trotz häufiger Autoklaviervorgänge bei einer Temperatur von 134°C über einen Zeitraum von 7 min bei einem Druck von 2,3 bar und bei ca. 95 % Luftfeuchte (gesättigter Wasserdampf) unbeschädigt bleibt, und bei dem insbesondere die CCD-Chip-Einheit bzw. deren einzelne Bestandteile nicht durch die Ausdehnungsbelastungen beschädigt werden.

Diese Aufgabe wird bei dem eingangs beschriebenen elektronischen Endoskop dadurch gelöst, daß die CCD-Chip-Einheit am distalen Ende eines CCD-Rohres elastisch gehalten ist, wobei das distale Ende des CCD-Rohres elastische Zungen aufweist, zwischen denen die CCD-Chip-Einheit (20) angeordnet ist.

Aufgrund dieser speziellen Halterung der CCD-Chip-Einheit bzw. deren einzelner Bestandteile können sich diese bei Durchführung des Autoklaviervorganges unterschiedlich ausdehnen, aber nach dem Autoklavieren wieder in die vorbestimmte Position, insbesondere auch gegenüber der Objektiveinheit des Endoskopes, zurückkehren, ohne durch diesen Vorgang Schäden zu erleiden.

In weiterer vorteilhafter Ausgestaltung des Endoskopes weist der Schaft unterschiedliche, jeweils einander angepaßte Durchmesser aufweisende und ineinandergeschobene und gegebenenfalls gegeneinander verschiebbare Rohre auf, von denen ein Rohr das die CCD-Chip-Einheit an seinem distalen Ende elastisch haltendes CCD-Rohr ist, in welchem auch die Videosignale leitenden Kabel verlaufen, ein weiteres Rohr ein einen größeren Durchmesser aufweisendes Objektiv-Rohr ist, an dessen distalem Ende die Objektiv-Einheit angeordnet ist und in welchem das CCD-Rohr längsverschieblich gelagert ist, und ein drittes Rohr ein einen wiederum größeren Durchmesser aufweisendes Mantelrohr zur Aufnahme des CCD-Rohres und des Objektiv-Rohres mit einem an seinem distalen Ende angeordneten Abschlußglas ist.
Durch diese Aufteilung des Innenraumes des Schaftes des Endoskopes können die einzelnen, am distalen Ende des Endoskopes angeordneten Bauteile wie CCD-Chip-Einheit, Objektiv-Einheit und das diese abschließende plane Abschlußglas in jeweils verschiedenen Rohren befestigt werden, die dann entsprechend den einzelnen Notwendigkeiten ausgebildet werden können. Die Ausbildung dieser drei vorgenannten Rohre gewährleistet, daß sie in ihrer Gesamtheit gegenüber dem von einem äußeren Rohr gebildeten Schaft des Endoskopes und auch gegenüber dem Gehäuse des Endoskopes vollständig dicht gegen Eindringen von Luft und Feuchtigkeit ausgebildet sind und auch gegenüber dem zwischen dem Mantelrohr einerseits und dem von diesem mit dem Schaft gebildeten ringförmigen Zwischenraum, der die zur Lichtübertragung dienenden Lichtleitfasern aufnimmt, abgeschlossen ist.

Dazu ist das Abschlußglas am distalen Ende des Mantelrohres mittels eines speziellen Lotes bei 800°C im Hochvakuum fest und dicht einlötet.

In vorteilhafter Weiterbildung der Erfindung ist das Mantelrohr an seinem proximalen Ende als Verbindungsbuchse ausgebildet und diese ist in einer Gehäusebuchse befestigt, die am proximalen Ende des Mantelrohres ausgebildet ist, wobei die Gehäusebuchse mit einem keramischen Steckbuchsensystem dicht nach außen abgeschlossen ist.

Schließlich ist vorgesehen, daß die einzelnen Oberflächen der Bauteile der CCD-Chip-Einheit jeweils mit einer Anti-Reflex-Mittel-Beschichtung ausgestattet sind.

Ein die Erfindung nicht beschränkendes Ausführungsbeispiel des vollautoklavierbaren Endoskopes wird anhand der nachstehend näher bezeichneten Zeichnungen und Figuren erläutert.

Es zeigen
- Fig. 1: eine Gesamtzusammenstellung mit den Abbildungen a) bis i), die die Bestandteile des Endoskopes einzeln wie auch in ihrer jeweiligen Zusammensetzung zeigen,

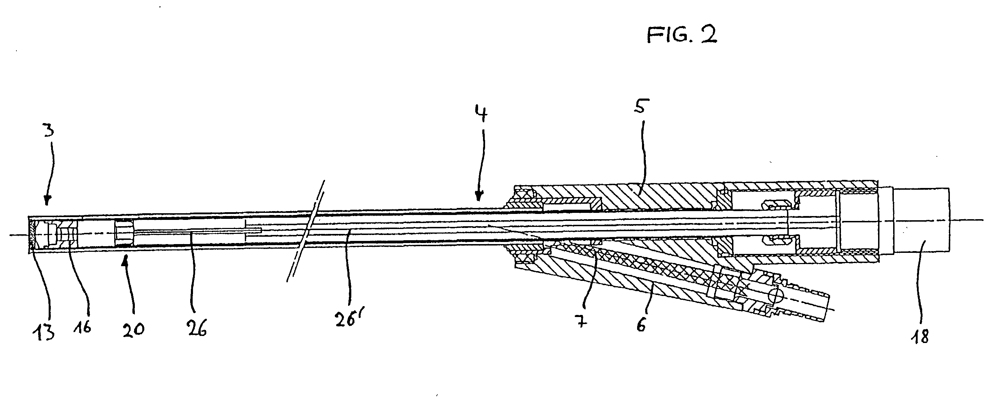

- Fig. 2: einen Schnitt durch das vollständig zusammengesetzte autoklavierbare Endoskop gemäß Abbildung a) in Fig. 1,
- Fig. 3: einen Schnitt durch den Schaft und das Gehäuse des Endoskopes gemäß Abbildung b) in Fig. 1,
- Fig. 4: einen Schnitt durch die autoklavierbare CCD- und Optik-Einheit gemäß Abbildung c) in Fig. 1, zusammengesetzt aus den in den Abbildungen b), e), g) und h) gezeigten weiteren Bestandteilen des Endoskopes,
- Fig. 5: einen Schnitt durch das Mantelrohr gemäß Abbildung d) in Fig. 1,
- Fig. 6: einen Schnitt durch das Objektivrohr mit Objektiv-Einheit gemäß Abbildung e) in Fig. 1,
- Fig. 7: einen Schnitt gemäß Abbildung f) in Fig. 1 betreffend den aus dem in Abbildungen g) und h) gezeigten CCD-Rohr und CCD-Chip zusammengesetzten Bauteil mit Verbindungsbuchse,
- Fig. 8: einen Schnitt durch das CCD-Rohr,
- Fig. 9: einen vergrößerten Schnitt durch die vollständige CCD-Chip-Einheit gemäß Abbildung h) in Fig. 1 und
- Fig. 10: ein vergrößertes Detail gemäß Abbildung i) in Fig. 1 gemäß Fig. 9.

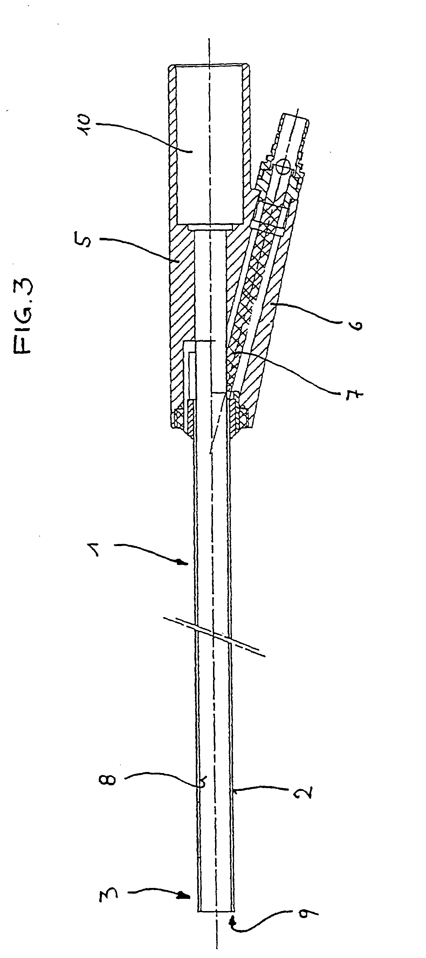

Das in den Figuren dargestellte voll-autoklavierbare Endoskop ist insbesondere für den Einsatz im medizinischen Bereich vorgesehen und besteht zunächst aus einem den Schaft 1 bildenden äußeren Rohr 2, das ein distales Ende 3 und ein proximales Ende 4 aufweist, welches in einem Gehäuse 5 gehalten ist. Das Gehäuse 5 selbst, wie insbesondere der Fig. 3 zu entnehmen ist, weist einen Anschluß 6 für das Lichtleiter-Glasfasersystem an einer externen Lichtquelle auf. In diesem Anschluß 6 sind die lichtleitenden Glasfasern 7 angeordnet, die im Gehäuse 5 in den Schaft 1 eingeführt werden, und zwar dergestalt, daß sie einen zwischen dem äußeren Rohr 2 und einem in diesem angeordneten und einen geringeren Durchmesser aufweisenden weiteren Rohr 8 gebildeten Zwischenraum 9 ausfüllen und von dem Gehäuse 5 bis zu dem distalen Ende 3 des Schaftes 1 geführt sind, damit dort das notwendige Licht zur Ausleuchtung des zu beobachtenden Bereiches austreten kann. Das Gehäuse 5 wiederum weist an seinem proximalen Ende eine Aufbohrung 10 auf, die zur Aufnahme entsprechender, auf den proximalen Enden der weiter unten näher beschriebenen und in den in den Schaft 1 des Endoskopes einzusetzender weiterer Rohre ausgebildeten Vorrichtungen vorgesehen sind.

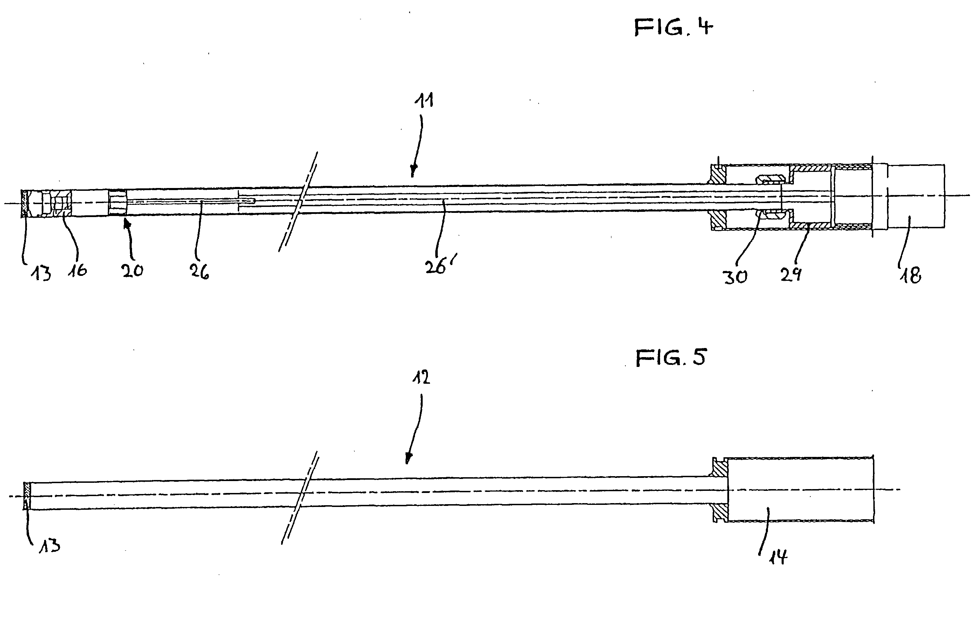

In diesen in Fig. 3 abgebildeten Schaft 1 wie in das Gehäuse 5 und die Aufbohrung 10 wird die in Fig. 4 dargestellte autoklavierbare CCD- und Optik-Einheit 11 vom proximalen Ende des Endoskopes her eingesteckt bzw. eingeschoben.

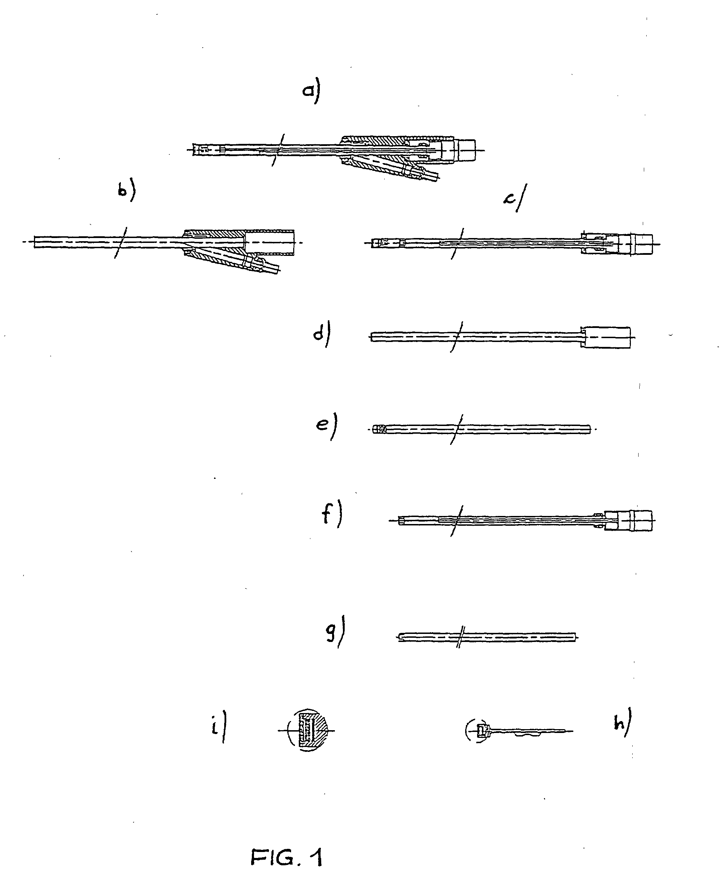

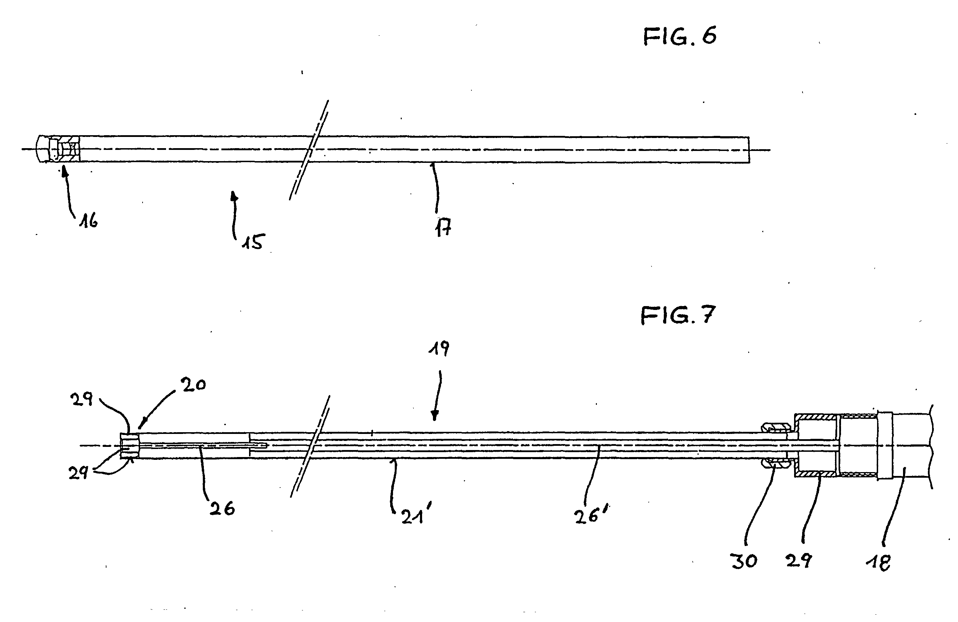

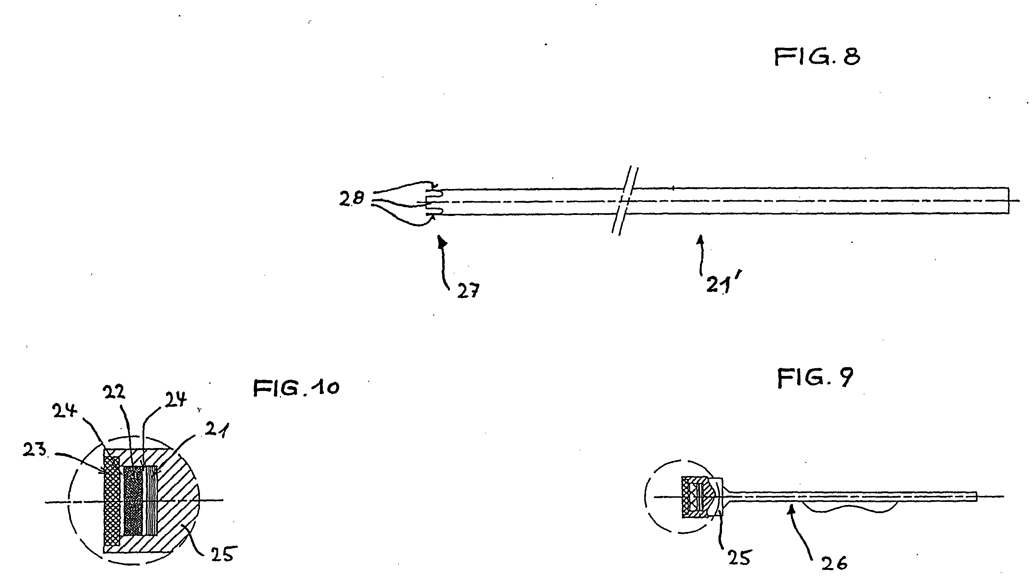

Diese in Fig. 4 dargestellte autoklavierbare Einheit 11 besteht im wesentlichen aus dem in Fig. 5 gezeigten Mantelrohr 12 mit Planglas 13 und Gehäusebuchse 14 sowie der darin eingesteckten und in Fig. 6 abgebildeten Objektiv-Einheit 15 mit Objektiv 16 und Objektivrohr 17 sowie der darin wiederum eingesteckten und in Fig. 7 gezeigten und mit einer Verbindungsbuchse 18 versehenen CCD-Chip-Rohr-Einheit 19 mit CCD-Chip-Einheit 20 und CCD-Rohr 21', die jeweils in den Figuren 8 und 9 dargestellt sind. Diese einzelnen Bauteile sind besonders übersichtlich hinsichtlich ihres Zusammenwirkens der Fig. 1 und den darin dargestellten Einzelabbildungen c) bis h) zu entnehmen.

Um den Aufbau der autoklavierbaren CCD- und Optik-Einheit 11 im weiteren näher zu erläutern, wird zunächst der Aufbau der CCD-Chip-Einheit 20 selbst und dann darauf aufbauend ihre Anordnung in dem CCD-Rohr 21' und weiter im Objektivrohr 17 und im Mantelrohr 12 beschrieben.

Die CCD-Chip-Einheit 20 ist in vergrößerter Darstellung gegenüber den anderen Figuren in Fig. 9 dargestellt, und gegenüber dieser Fig. 9 in wiederum vergrößerter Darstellung in Fig. 10.

Die CCD-Chip-Einheit 20 umfaßt im wesentlichen den CCD-Chip 21 selbst, dem ein IR-Cut-Filter 22 und diesem wiederum ein Kristallfilter 23 vorgeordnet sind, wobei zwischen Kristallfilter 23 und IR-Cut-Filter 22 und dem CCD-Chip 21 Zwischenräume 24 vorgesehen sind, die lediglich mit Luft angefüllt sind.

Diese einzelnen Bestandteile werden von einer Fassung 25 gehalten, an der auch die von der CCD-Chip-Einheit 20 erarbeiteten Video-Signale weiterleitenden elektrischen Kabel 26' enthaltende Platine 26 befestigt ist.

Diese speziell in Fig. 9 dargestellte CCD-Chip-Einheit 20 ist in dem in Fig. 8 abgebildeten CCD-Rohr 21', und zwar an dessen distalem Ende 27 gehalten, das elastische Zungen 28 aufweist. Dieser Zusammenbau ist im weiteren der Fig. 7 zu entnehmen, dort wird auch die Anordnung des CCD-Rohres 21' in der an seinem proximalen Ende vorgesehenen Verbindungsbuchse 18 gezeigt, die mittels einer Rohrklemmbuchse 29 und einer Spannschraube 30 erfolgt.

Durch den getrennten Aufbau der einzelnen vorbeschriebenen Teile der CCD-Chip-Einheit 20 in dem Silicium-Substrat 25 und durch die Befestigung der CCD-Chip-Einheit 20 zwischen den Zungen 28 des distalen Endes des CCD-Rohres 21 kann sich die CCD-Chip-Einheit 20 entsprechend den sich gerade bei dem Autoklavierprozeß und der anschließenden Abkühlung verändernden Temperaturen hinsichtlich ihrer unterschiedlichen Ausdehnungen entsprechend verhalten, wobei die Zungen 28 dafür Sorge tragen, daß sich die CCD-Chip-Einheit 20 bei Normaltemperatur wieder in den vorbestimmten Bereich und der in der vorbestimmten Anordnung befindet.

Die in Fig. 7 dargestellte CCD-Chip-Rohr-Einheit 19, bestehend aus CCD-Rohr 21' mit Verbindungsbuchse 18, wird von der proximalen Seite her in die in Fig. 6 dargestellte Objektiveinheit 15 eingeschoben. Diese Objektiveinheit 15 besteht im wesentlichen aus dem in Fig. 6 gezeigten Objektivrohr 17 und der an dessen distalem Ende angeordneten Objektiv-Einheit 16, die aus einzelnen Objektiv-Bestandteilen zusammengesetzt ist.

Dieses vorbeschriebene Endoskop ist voll autoklavierbar, und zwar aufgrund seines besonderen Aufbaus der CCD-Chip-Einheit 20 und wiederum dessen spezieller Anordnung in einem CCD-Rohr 21', das, eingesteckt in das Objektiv-Rohr 17, in dem Mantelrohr 12 gehalten ist, und, mit diesem zusammen, durch die dichte Ausgestaltung des am distalen Ende angeordneten Planglases 13 und der ebenfalls dicht ausgebildeten Gehäusebuchse 14 in Verbindung mit der Rohrklemmbuchse 29 und der Verbindungsbuchse 18, die mittels eines hermetisch dichten Steckbuchsensystems nach außen abgeschlossen ist.

Diese vorbeschriebene Einheit bildet die eigentliche vollautoklavierbare CCD- und Objektiv-Einheit, die von dem Schaft 1 und dem Gehäuse 5 des Endoskopes eingeschlossen wird.

Im übrigen erlaubt der Einbau der CCD-Chip-Einheit 20 zwischen elastischen Zungen 28 am distalen Ende 27 des CCD-Rohres 21' und durch die Verschiebbarkeit dieses CCD-Rohres 21' im Objektiv-Rohr 17, die CCD-Chip-Einheit 20 in einfacher Weise aus der CCD-Chip-Rohr-Einheit 19 herauszuziehen und ggfs. durch eine neue Einheit zu ersetzen.

## Patentansprüche

1. Elektronisches Endoskop mit einem Halbleiter-Bildsensor in Form eines CCD-Chips (21) und einer Filter-Anordnung (22, 23), die mit dem CCD-Chip (21) zu einer CCD-Chip-Einheit (20) zusammengefaßt ist, zur Aufnahme der von einem Objektiv (16) aufgenommenen Bilder und einer elektronischen Schaltung, wobei das Endoskop im wesentlichen aus einem Schaft (1) mit einem distalen und einem proximalen Ende (3 bzw. 4) besteht und das Objektiv (16) sowie die CCD-Chip-Einheit (20) am distalen Ende (3) angeordnet und das proximale Ende (4) in einem den Schaft (1) haltenden und einen Glasfaserlichtanschluß (6) umfassenden Gehäuse (5) gehalten ist, wobei die Bestandteile der hinter dem Objektiv (16) angeordneten CCD-Chip-Einheit (20), nämlich ein Kristallfilter (23), ein IR-Cut-Filter (22) und der CCD-Chip (21) jeweils mit einem freien Zwischenraum zueinander angeordnet und in einer Metallfassung (25) gehalten sind, die einen Bestandteil der Einheit bildet und an welche sich eine Platine (26) mit den die Videosignale leitenden Kabeln (26') anschließt, **dadurch gekennzeichnet, daß** die CCD-Chip-Einheit (20) am distalen Ende (27) eines CCD-Rohres (21') elastisch gehalten ist, wobei das distale Ende (27) des CCD-Rohres (21') elastische Zungen (28) aufweist, zwischen denen die CCD-Chip-Einheit (20) angeordnet ist.

2. Elektronisches Endoskop nach Anspruch 1, **dadurch gekennzeichnet, daß** der Schaft (1) des Endoskopes unterschiedliche, jeweils geringe, aneinander angepaßte Durchmesser aufweisende und ineinander geschobene und ggfs. gegeneinander verschiebbare Rohre (12, 17 und 21') aufweist, von denen das Rohr (21') ein die CCD-Chip-Einheit (20) an seinem distalen Ende (27) elastisch haltendes CCD-Rohr (21') ist, in welchem auch die die Videosignale leitenden Kabel (26') aufnehmende Platine (26) einsteht, das Rohr (17) ein einen größeren Durchmesser aufweisendes Objektiv-Rohr (17) mit an seinem distalen Ende angeordnetes Objektiv (16) ist, in welchem das CCD-Rohr (21') längsverschieblich gehalten ist, und das Rohr (12') ein wiederum einen größeren Durchmesser aufweisendes Mantelrohr (12) zur Aufnahme des CCD-Rohres (21) und des Objektiv-Rohres (17) mit einem an seinem distalen Ende angeordneten planen Abschlußglas (13) ist.

3. Elektronisches Endoskop nach Anspruch 2, **dadurch gekennzeichnet, daß** das plane Abschlußglas (13) dicht im Mantelrohr (12) befestigt ist.

4. Elektrisches Endoskop nach Anspruch 3, **dadurch gekennzeichnet, daß** das Abschlußglas (13) am distalen Ende des Mantelrohres (12) mittels eines speziellen Lotes bei 800°C im Hochvakuum fest und dicht eingelötet ist.

5. Elektronisches Endoskop nach Anspruch 2, **dadurch gekennzeichnet, daß** das Mantelrohr (12) an seinem proximalen Ende eine Gehäusebuchse (14) aufweist, in welche eine Rohrklemmbuchse (29) einsteht, die über eine Spannschraube (3) mit dem CCD-Rohr (21') verbunden ist und wobei in der Rohrklemmbuchse (29) eine eine hermetisch dichte Steckverbindung aufweisende Verbindungsbuchse (18) angeordnet ist, die den Abschluß des proximalen Endes des Mantelrohres (12) bildet.

6. Elektronisches Endoskop nach einem der vorstehenden Ansprüche, **dadurch gekennzeichnet, daß** die Oberflächen der Bauteile (21, 22 und 23) der CCD-Chip-Einheit (20) jeweils mit einer Anti-Reflexmittel-Beschichtung versehen sind.

## Claims

1. Electronic endoscope with a semiconductor image sensorin the form of a CCD chip (21) and a filter arrangement (22, 23) which is combined with the CCD chip (21) to form a CCD chip unit (20) to accept the pictures taken by a lens (16) and an electronic circuit whereby the endoscope substantially comprises a shaft (1) with a distal and a proximal end (3 or 4) and the lens (16) and the CCD chip unit (20) are arranged at the distal end (3) and the proximal end (4) is held in a housing (5) holding the shaft (1) and encompassing a glass fibre light connector (6) whereby the components of the CCD chip unit (20), namely a crystal filter (23), an IR cut filter (22) and the CCD chip (21), arranged behind the lens (16) are each arranged with a free interspace in relation to each other and held in a metal holder (25) which forms a component of the unit and to which is connected a board (26) with the cables (26') conducting the video signals, **characterised in that** the CCD chip unit (20) is held elastically at the distal end (27) of the CCD tube (21') whereby the distal end (27) of the CCD tube (21') has elastic tongues (28) between which the CCD chip unit (20) is arranged.

2. Electronic endoscope according to claim 1, **characterised in that** the shaft (1) of the endoscope has different tubes (12, 17 and 21') which are each small and have diameters matched to each other and are telescoped inside each other and are optionally relocatable in relation to each other, of which the tube (21') is a CCD tube (21') holding the CCD chip unit (20) elastically at its distal end (27), in which the board (26) holding the cables (26') transmitting the video signals is also inserted, the tube (17) is a lens tube (17) with a larger diameter with a lens (16) arranged at its distal end in which the CCD tube (21') is held in a longitudinally displaceable manner and the tube (12') is a jacket tube (12) again with a larger diameter to accept the CCD tube (21) and the lens tube (17) with a flat glass cover (13) arranged at its distal end.

3. Electronic endoscope according to claim 2, **characterised in that** the flat glass cover (13) is tightly secured in the jacket tube (12).

4. Electric endoscope according to claim 3, **characterised in that** the glass cover (13) is firmly and tightly soldered to the distal end of the jacket tube (12) by means of a special solder at 800 °C in a high vacuum.

5. Electronic endoscope according to claim 2, **characterised in that** the jacket tube (12) has a housing bush (14) at its proximal end in which a tube clamp bush (29) is inserted which is connected to the CCD tube (21') by means of a clamping bolt (3) and whereby arranged in the tube clamp bush (29) is a connecting bush (18) with a hermetically sealed plug-and-socket connection with said connecting bush forming the termination of the proximal end of the jacket tube (12).

6. Electronic endoscope according to any one of the above claims, **characterised in that** the surfaces of the components (21, 22 and 23) of the CCD chip unit (20) are each provided with an antireflex coating.

## Revendications

1. Endoscope électronique comportant un capteur d'image à semiconducteurs sous la forme d'une puce CCD (21), ainsi qu'un agencement de filtres (22, 23), qui est réuni à la puce CCD (21) pour former une unité de puce CCD (20), pour recevoir les images prises par un objectif (16) et un circuit électronique, l'endoscope étant essentiellement constitué d'une tige (1) avec une extrémité distale (3) et une extrémité proximale (4), et l'objectif (16) ainsi que l'unité de puce CCD étant disposés à l'extrémité distale (3) et l'extrémité proximale (4) étant maintenue dans un boîtier (5) maintenant la tige (1) et entourant une prise de lumière à fibres optiques de verre, les composants de l'unité de puce CCD (20), disposés derrière l'objectif (16), à savoir un filtre cristal (23), un filtre IR-cut (22) et la puce CCD (21), étant disposés avec un espace libre entre eux et étant maintenus dans une monture métallique (25) qui forme une partie intégrante de l'unité et à laquelle se raccorde une platine (26) avec les câbles (26') guidant les signaux vidéo, **caractérisé en ce que** l'unité de puce CCD (20) est maintenue élastiquement à l'extrémité distale (27) d'un tube CCD (21'), l'extrémité distale (27) du tube CCD (21') présentant des languettes élastiques (28) entre lesquelles est disposée l'unité de puce CCD (20).

2. Endoscope électronique selon la revendication 1, **caractérisé en ce que** la tige (1) de l'endoscope comporte différents tubes (12, 17 et 21') présentant chacun de petits diamètres adaptés les uns aux autres, enfilés les uns dans les autres et pouvant éventuellement coulisser les uns par rapport aux autres, dont le tube (21') est un tube CCD (21') maintenant élastiquement l'unité de puce CCD (20) à son extrémité distale (27), dans lequel se trouve aussi la platine (26) recevant les câbles (26') guidant les signaux vidéo, le tube (17) est un tube objectif (17), présentant un diamètre plus grand avec objectif (16) disposé à son extrémité distale, dans lequel le tube CCD (21') est maintenu de manière à pouvoir coulisser longitudinalement, et le tube (12') est un tube d'enveloppe (12), présentant à nouveau un plus grand diamètre, destiné à recevoir le tube CCD (21) et le tube objectif (17) avec un verre de terminaison (13) plan disposé à son extrémité distale.

3. Endoscope électronique selon la revendication 2, **caractérisé en ce que** le tube de terminaison (13) plan est fixé de manière étanche dans le tube d'enveloppe (12).

4. Endoscope électronique selon la revendication 3, **caractérisé en ce que** le verre de terminaison (13) est brasé de manière fixe et étanche à l'extrémité distale du tube d'enveloppe (12), au moyen d'un métal de brasage spécial à 800 °C, sous vide poussé.

5. Endoscope électronique selon la revendication 2, **caractérisé en ce que** le tube d'enveloppe (12) comporte, à son extrémité proximale, une douille de boîtier (14) dans laquelle se trouve une douille de serrage de tube (29) qui est reliée, par une vis de serrage (3), au tube CCD (21'), et dans la douille de serrage de tube (29) étant disposée une douille de liaison (18) comportant une liaison par enfichage hermétiquement étanche, laquelle forme la terminaison de l'extrémité proximale du tube d'enveloppe (12).

6. Endoscope électronique selon l'une des revendications précédentes, **caractérisé en ce que** les surfaces des composants (21, 22 et 23) de l'unité de puce CCD (20) sont pourvues chacune d'un revêtement antireflet.
